# EUROPEAN PATENT SPECIFICATION

(11) **EP 2 036 511 B1**
(45) Date of publication and mention of the grant of the patent: **07.09.2011**
(21) Application number: 08015839.7
(22) Date of filing: 09.09.2008
(51) Int. Cl.: A61B 18/14

(54) **Electrosurgical instrument**
Elektrochirurgisches Instrument
Instrument électro-chirurgical

(30) Priority: 13.09.2007 US 900715
(43) Date of publication of application: 18.03.2009
(73) Proprietor: Tyco Healthcare Group, LP, North Haven CT 06473 (US)
(72) Inventor: Arts, Gene H., Berthoud, CO 80513 (US)
(74) Representative: HOFFMANN EITLE

(56) References cited:
- DE-A1- 19 524 645
- US-A- 4 781 175
- US-A- 5 707 402
- US-A1- 2001 051 805

## Description

### BACKGROUND

### Technical Field

The present invention relates to devices for use in open, laparoscopic or endoscopic procedures for treating tissue. More particularly, in one embodiment, the present disclosure relates to gas-enhanced surgical instruments including a pneumatic tissue dissector.

### Background of Related Art

While it is appreciated that the present disclosure relates to devices for use during open and closed, laparoscopic and endoscopic procedures, the focus of the discussion will be on devices for use mainly during closed procedures. The less invasive, laparoscopic and endoscopic procedures, are conducted through small incisions in the body. Unlike during open procedures, space for manipulating surgical devices during closed procedures is limited. Surgical techniques which are routine in open procedures are often more difficult to perform using laparoscopic and endoscopic instruments and techniques.

One such surgical technique is tissue manipulation where limited access and space may make tissue manipulation extremely challenging. Pneumatic dissectors have been developed to address the issue of limited space with regards to instrument manipulation. Pneumatic dissectors use gas, e.g. CO₂, to divide tissue plains rather then tearing at the tissue with a grasping device or cutting through tissue with a scalpel. A benefit of using this technique to separate tissue, as opposed to tearing or cutting, is reduced trauma to the tissue. Even with the less evasive pneumatic dissecting, some damage still does occur to the tissue which results in bleeding. The bleeding must be stopped before the surgery can be completed, therefore, another instrument is necessary to seal the tissue.

Gas-enhanced surgical instruments for coagulating tissue are well known in the art. U.S. publication No. 2006-0052772 entitled "GAS-ENHANCED SURGICAL INSTRUMENT" filed September 19, 2005, teaches one such a device. In a gas-enhanced electrosurgical instrument an ionizable gas, e.g. argon, is forced from a gas supply through the instrument and ionized by an electrode prior to being emitted from the distal end of the instrument. The gas supply may be self-contained and/or selectively replaceable, or may be remotely supplied. The ionized gas exiting the distal end of the instrument typically flows at a rate of less than about 1 liter/minute. Providing the gas at this flow rate is believed to effectively cloud the tissue area and create an ionizable gas "atmosphere" to gently coagulate the tissue. Gas-enhanced surgical instruments are very useful in laparoscopic and endoscopic procedures because of the limited operational manipulation necessary. Hemostasis can also be controlled without touching the tissue or without risk of fouling the electrode tip or excess thermal damage to the tissue.

Tissue separated by a pneumatic dissector may be require coagulation or cauterization many times throughout a surgical procedure. A surgeon is thus required to alternate between using the pneumatic dissector and the electrosurgical instrument. While somewhat routine during an open procedure, alternating between the pneumatic dissector and the electrosurgical instrument is complicated during laparoscopic and endoscopic procedures where space is limited and operating multiple instruments at once is difficult at best. When only one instrument can be used at a time, the surgeon must completely remove one instrument before replacing it with the other. The constant alternating between dissector and coagulator also increases the length of time to perform the surgery.

DE 195 24 645, US 5 707 402, US 4 781 175, US 2001/0051805 disclose electrosurgical instruments use a source of gas. The preamble of claim 1 is based on DE 195 24 645, which discloses electromagnetic values for controlling the flow rate of the gas.

### SUMMARY

The present invention provides an electrosurgical instrument as defined in claim 1 comprising a housing including a tube extending therethrough, the tube having proximal and distal ends, the proximal end being adapted to connect to at least a first source of gas and the distal end being configured to deliver gas to a surgical site, and an actuator configured to selectively regulate the flow of gas through the tube, the actuator having at least a first position which allows a first predetermined rate of gas to flow through the tube for a first surgical purpose, and at least one subsequent position which allows at least one different rate of gas to flow through the tube for at least a second surgical purpose.

The electrosurgical instrument may further include an electrode assembly configured to selectively ionize the gas for at least one of the first or second surgical purposes. The actuator is configured to prevent the flow of gas through the tube.

The first source of gas may be a portable cartridge or a cylinder containing pressurized ionizable gas.

The electrosurgical instrument may further include at least one pressure relief mechanism operatively coupled to the tube. The pressure relief mechanism may be located in close proximity to the patient. The first pressure relief mechanism is operatively coupled to the tube for regulating gas pressure within the tube and a second pressure relief mechanism operative coupled to the hand-held device for regulating gas within the surgical site. The pressure relief mechanism may regulate the pressure flowing through the tube to below 50mmHg.

The electrosurgical instrument may comprise a first pressure relief mechanism operatively coupled to a first part of the tube and a second pressure relief mechanism operatively coupled to a second part of the tube proximal to the first pressure relief mechanism.

Also provided is an electrosurgical system as defined in claim for dissecting and coagulating tissue, comprising an electrosurgical generator, at least a first source of gas, and a hand-held applicator comprising a housing including a tube extending therethrough, the tube having proximal and distal ends, the proximal end being adapted to connect to the at least first source of gas and the distal end being configured to deliver gas to a surgical site, an actuator configured to selectively regulate the flow of gas through the distal end of the tube, the actuator having at least a first position which allows a first predetermined rate of gas to flow for a first surgical purpose, and at least one subsequent position which allows a different rate of gas to flow through the tube for a second surgical purpose, and an electrode mounted in proximity to the distal end of the tube, the electrode operably connected to the electrosurgical generator and configured to selectively ionize the gas passing thereby.

The electrosurgical instrument maycomprise an electrode mounted in proximity to the distal end of the tube, the electrode operably connected to the electrosurgical generator and configured to selectively ionize the gas passing thereby; and at least one pressure relief mechanism operably associated with the tube.

### BRIEF DESCRIPTION OF THE DRAWINGS

The foregoing summary, as well as the following detailed description will be better understood when read in conjunction with the appended figures. For the purpose of illustrating the present disclosure, one particular embodiment is shown. It is understood, however, that the present disclosure is not limited to the precise arrangement and instrumentalities shown.

FIG. 1 is a cross-sectional side view of the gas-enhanced surgical device according an embodiment of the present invention;

FIG. 2 is a schematic view of the gas-enhanced surgical device of FIG. 1, operated as a pneumatic tissue dissector;

FIG. 3 is a schematic view of the gas-enhanced surgical device of FIGS. 1 and 2, operated as a tissue coagulator; and

FIG. 4 is a schematic view of an alternate gas-enhanced surgical device that is not an embodiment of the present invention; and

FIG. 5 is a schematic view of another gas-enhanced surgical device that is not an embodiment of the present invention.

### DETAILED DESCRIPTION

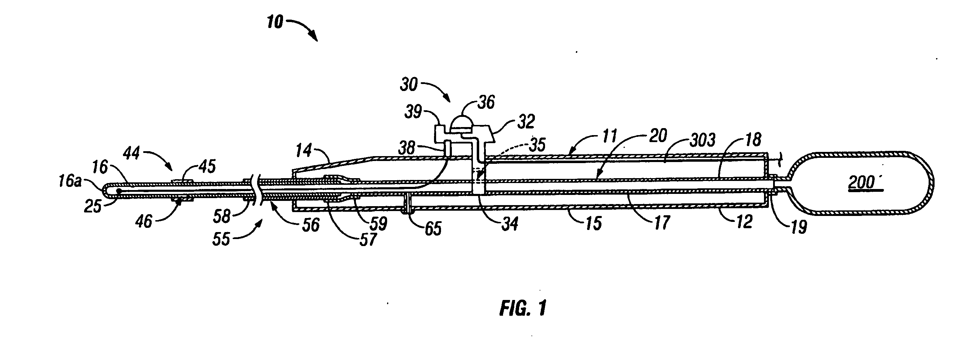

A particular embodiment of a gas-enhanced electrosurgical device having a pneumatic dissector in accordance with an embodiment of the present invention is described in detail below with reference to the drawing figures 1 to 3 wherein like reference numerals identify similar or identical structural elements.

This application discloses various electrosurgical devices that are adapted for use as a pneumatic tissue dissector and as a tissue coagulator. The electrosurgical device may have a self-contained or remote source of pressurized ionizable gas and may be used for various surgical functions, including arresting bleeding tissue, desiccating surface tissue, eradicating cysts, forming eschars on tumors, thermically marking tissue and pneumatically dissecting tissue. For ease of description, the instrument described herein is configured for use as a pneumatic dissector for separating tissue and as a coagulator for sealing bleeding tissue. However, those skilled in the art will appreciate that certain modifications can be made to the electrosurgical device of the present disclosure so that the device can perform other surgical functions without departing from the scope of this disclosure. Moreover, while it is preferable to use argon as the ionizable gas for promulgating coagulation of tissue, for other surgical functions another ionizable gas or a combination of ionizable gases may be utilized to achieve the desired result.

Referring to FIGS. 1-3, an embodiment of the presently invention is illustrated and generally designated as electrosurgical device 10. Although the basic operating features of electrosurgical device 10 for use in closed procedures are described herein, the same or similar operating features may be employed on or used in connection with an electrosurgical device for use in open procedures, manually or robotically operated, without departing from the scope of the present disclosure. The term "electrosurgical energy" herein refers to any type of electrical energy which may be utilized for medical procedures.

As shown in FIG. 1, device 10 includes a frame, shown as an elongated housing 11 having a proximal end 12, a distal end 14 and an elongated body portion 15 extending therethrough for supporting and/or housing a plurality of internal and/or external mechanical and electromechanical components thereon and therein. In this disclosure, as is traditional, the term "proximal" will refer to the end of device 10 (or other element) which is closer to the user, while the term "distal" will refer to the end which is further from the user.

An elongated gas supply channel or tube 20 is defined in housing 11 and runs generally longitudinally therethrough. Tube 20 includes a distal end 16, a proximal end 18 and a middle portion 17, therebetween. Middle portion 17 of tube 20 extends through distal end 14 of housing 11 and includes a length which may vary depending on a particular surgical application, e.g., a longer middle portion 17 may be required for accessing deeper with a body cavity. Tube 20 includes one or more sections of tubing composed of one or more different materials such as metal, plastic, polymers or the like. Tube 20 is also dimensioned to include a diameter sufficiently large enough to permit the free flow of ionizable gas therethrough.

The distal end 16 of tube 20 includes a port 16a defined therein which is configured to emit, expel or disperse gas traveling through tube 20. Preferably, the distal end 16 of tube 20 includes one or more smooth or rounded outer surfaces such that the distal end 16 does not damage tissue that may be incidentally contacted during the use of device 10. Distal end 16 may be configured to facilitate or promote the dispersion of the ionized gas from distal port 16a in a uniform and consistent manner. For example, distal end 16 may be tapered on one, both or all sides thereof to direct the ionized gas toward a surgical or operative site 75. Alternatively, distal port 16a may be configured to disrupt or agitate the dispersion or flow of the ionized gas exiting distal port 16a to enhance coagulation by creating a more turbulent gas flow. It is contemplated that many suitable devices, e.g., screws, fans, ribbon, blades, helical oscillators, baffles, or other types of flow agitators may be employed to cause the gas to flow more or less turbulently or with other predetermined flow characteristics through tube 20 and/or out of distal port 16a. It is further envisioned that distal port 16a or distal end 16 may be configured to increase air flow during tissue dissection.

Proximal end 18 of tube 20 includes a proximal port 19 at an end thereof which extends through proximal end 12 of housing 11. Proximal end 18 of tube 20 is adapted to operatively connect to a source of pressurized gas 200 via interface 202, e.g., hose, tube, or other mechanically connectable element. The pressurized gas source may be a cylinder or a portable cartridge 200' which operatively attached to the device 10, e.g., the proximal end 12 of housing 11 may be configured to operatively retain a pressurized gas cartridge (not shown). In this embodiment, proximal port 19 may be configured to operably connect with the pressurized gas cylinder.

During use as a dissector, tube 20 of device 10 supplies a steady stream of pressurized gas through distal port 16a. Alternately, when using device 10 as a coagulator, tube 20 of electrosurgical device 10 supplies a gentle flow of pressurized gas to the proximity of an active electrode 25 located adjacent distal end 16 of tube 20. Electrode 25 is located proximal of port 16a such that the gas emitted from distal port 16a may be ionized as it passes electrode 25. Highly pressurized, non-ionized gas is directed out of distal port 16a of tube 20 when device 10 is used as a dissector. Low pressure, ionized gas flows from distal port 16a when device 10 is operated as a coagulator. The flow rate of the pressurized gas may be adjusted using a pressure regulator (not shown). The gas flow rate employed is dependent upon factors such as the instrument being used and/or the type of surgery or procedure being performed.

Electrosurgical device 10 also includes at least one actuator, e.g., a dial, button, lever, switch or other suitable element, generally designated 30, for actuating and/or selectively adjusting the flow of pressurized gas from pressurized gas source 200 thorough port 16a. Actuator 30 (or second actuator) may also be used to actuate and selectively adjust the delivery of electrosurgical energy from the energy source, i.e., from generator 300, to the active electrode 25 for ionizing the inert gas for use at surgical site 75. Actuator 30 may be supported atop housing 11 to activate the electrode 25 and/or adjust the flow rate of the gas. More particularly, actuator 30 includes a base 32 which operatively connects to housing 11 and which includes a stem 34 which extends therefrom. Stem 34 operatively communicates with a lever 36 positioned on, in or about base 32 which switches device 10 between coagulating and dissecting modes. Actuator 30 further includes a tab 38 which is configured to lock and/or maintain the device 10 in a particular operating configuration, e.g., as a coagulator or dissector. A recess 39 defined in housing 11 may be included which mechanically interfaces with tab 38 to lock the device 10 in a particular operating configuration.

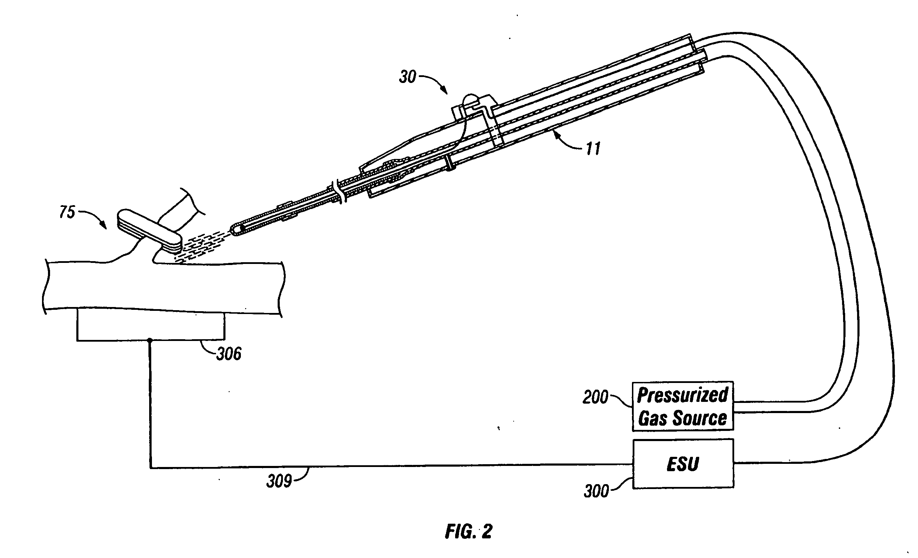

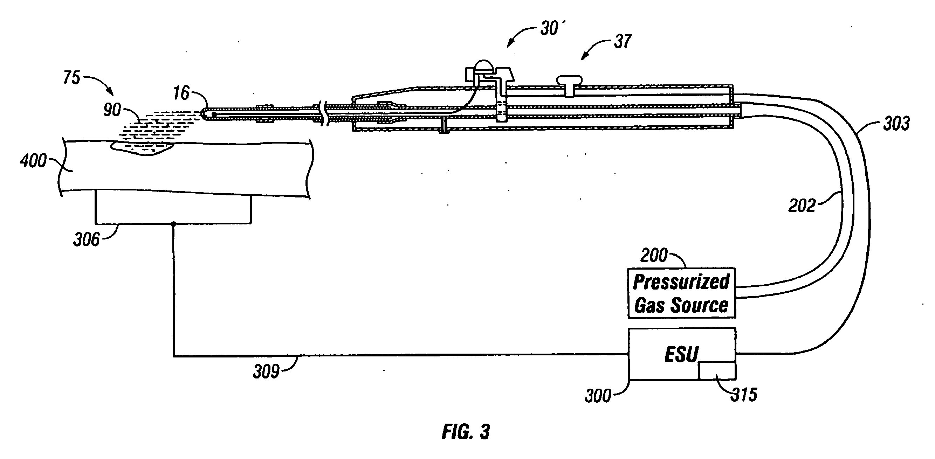

As shown and described in FIGS. 1-3, when lever 36 is in a first position (FIG. 2), device 10 operates as a dissector. When lever 36 is in a second position (FIG. 3), device 10 operates as a coagulator. Actuator 30 is further configured to receive an electrosurgical current from generator 300 via electrical cable 303. Actuator 30 is further configured to selectively deliver current to electrode 25 during the use of device 10 as a coagulator. During use as a dissector, no electrosurgical energy is delivered to active electrode 25.

According to the present invention and as shown in Figs. 1-3, stem 34 of actuator 30 includes an opening 35 defined therethrough which communicates with middle portion 17 of tube 20. Stem 34 is positionable by base 32 from a first position which closes off the flow of gas through tube 20 to a variety of intermediate or incremental positions which allow the flow of varying amounts of gas through tube 20. More particularly, the depression of the base 32 of the actuator 30 causes the movement of stem 34 and opening 35. The more base 32 is depressed, the less obstructed is the flow of gas through tube 20. Upon complete depression of base 32 opening 35 is completely aligned with tube 20 and the pressurized gas is permitted to freely flow through tube 20. Alternatively, base 32 may include one or more mechanical elements (not shown) which incrementally regulate the flow of gas through the tube, but this would not constitute an embodiment of the present invention. Indicia (not shown) may be included on actuator 30 to indicate the position of stem 34 and volume of gas flow through tube 20. Stem 34 may also be spring biased to return to the first position upon release of lever 36.

Actuator 30 may be configured with a valve system such that as stem 34 is depressed the valve opens and allows for the flow of gas through tube 20, but this would not constitute an embodiment of the present invention. Again, unobstructed flow of gas through tube 20 occurs when actuator 30 is completely depressed and the valve system is completely open. The valve system may be configured such that in dissecting mode the valve is permitted to completely open, while in coagulation mode the valve is only permitted to open to the extent necessary to provide enough gas for coagulating tissue. More or less gas may be applied depending on the amount stem 34 is depressed. It is envisioned that various other valves and valve systems may be used.

Alternately, stem 34 may be configured to pinch or squeeze tube 20 in order to regulate the flow of gas, but this would not constitute an embodiment of the present invention. Tube 20 may be configured to engage stem 34 such that in an inactive state, actuator 30 prevents the flow of gas through tube 20. The pinching system may operate in a manner similar to the previously described valve system. A fully depressed stem 34 completely releases or opens tube 20, thus allowing the free flow of pressurized gas for dissecting purposes while a partial depression only partially obstructs tube 20 to regulated flow.

As mentioned above, the relative position of lever 36 determines the extent that stem 34 may be depressed for coagulation and dissecting modes. It is also contemplated that lever 36 may be configured to provide a signal back to the energy source relating to the relative position of lever 36 to control energy distribution. More particularly, when lever 36 is in a first dissection position recess 39 formed in base 32 remains unobstructed by lever 36. In this manner, tab 38 may be completely received within recess 39 and stem 34 may be completely depressed within housing 11. When lever 36 is advanced into a second coagulation position, recess 39 formed in base 32 becomes obstructed by lever 36 and tab 38 can not be completely received within recess 39 which, in turn, prevents stem 34 from being completely depressed.

Actuator 30 is also configured to operatively communicate with active electrode 25 and couple to an electrical energy source 300. Electrosurgical energy, produced in generator 300 in transmitted through housing 11 via electrical cable 303. Electrical cable 303 may enter housing 11 at any location, preferably at proximal end 12. Electrosurgical energy may then passes through actuator 30 before traveling down electrical cable 303 through tube 20 and to active electrode 25. As mentioned above, the relative position of lever 26 may also regulate the energy distribution, i.e., activation of device 10 in a dissection mode does not transmit energy to electrode 25 while activation in a coagulation mode transmits energy to active electrode 25. In this manner, device 10 cannot accidentally ionize the high pressure gas being emitted from distal port 16 when being used as a dissector. As can be appreciated, the lever 36 may also be positionable to turn off the generator 300 when disposed in the dissection mode.

As shown in Fig. 3, the device 10 may alternatively include an actuator 30' which regulates the flow of the pressurized ionizable gas through the tube 20 and a switch 37 which controls the activation of electrode 25. Prior to exiting distal end 16 of tube 20, the gas is ionized to form a plasma cloud 90 which gently coagulates tissue.

In an alternate embodiment, after actuation of actuator 30 and initiation of gas flow to through distal port 16a, one or more controllers 315 (see Fig. 3) may be included to sequence or control the ignition of the electrode 25, e.g., delay ignition, either mechanically, electro-mechanically or utilizing delay circuitry or a delay algorithm (not shown). It is contemplated that providing the controller(s) 315 enhances the delivery of the ionized gas to operating site 75. As can be appreciated, the delay circuitry or algorithm may be incorporated in actuator 30 or generator 300.

As shown in FIGS. 2 and 3, and as in most monopolar electrosurgical systems, a return electrode or pad 306 is typically positioned under the patient and connected to a different electrical potential on electrosurgical generator 300 via cable 309. During activation, return pad 306 acts as an electrical return for the electrosurgical energy emanating from electrosurgical device 10. It is envisioned that various types of electrosurgical generators 300 may be employed for use with electrosurgical device 10, such as those generators sold by Valleylab, Inc. - a division of Tyco Healthcare Group LP, of Boulder, Colorado.

It is also contemplated that actuator 30 (or generator 300) may cooperate with one or more sensors which can be attached to device 10, housing 11 and/or electrode 25 which continually measure or monitor a condition at operative site 75, e.g., the amount of tissue coagulation, and relays the information back to generator 300 or provides visual or audible feedback to the operator. For example, a control system or a safety circuit (not shown) may be employed which automatically (e.g., through a shut-off switch) reduces pressure or partially closes actuator 30 if an obstruction is indicated. Alternatively or in addition, the safety circuit may be configured to cut off the energy to tissue 400 and/or activate or release a pressure relief valve to release the pressure of the pressurized gas based upon a sensed condition (e.g., an embolic condition or concern) by a sensor or by the surgeon. Alternatively, a sensor may provide feedback to actuator 30 or generator 300 to optimize coagulation of the tissue 400 based upon distance from the tissue deduced from the measured back pressure in tube 20, based upon tissue type or based upon tissue response. Other sensors may be employed to measure the flow of gas through tube 20 and may be electrically connected to one or more flow regulators, e.g., actuator 30, to automatically regulate the flow of gas though tube 20 and passed electrode 25.

As mentioned above and as shown in Fig. 1, electrosurgical device 10 may be configured with one or more safety mechanisms which are configured to prevent the build-up of excess pressure and reduce the chances of embolisms which are known to occur at pressures greater than 50 mmHg. For example, a first pressure relief mechanism 44 may be included which is configured to prevent the pressure build-up through the device. First pressure relief mechanism 44 includes an elastic band 45 positioned about an opening 46 defined within a distal portion of tube 20. Preferably, opening 46 is located within the section of tube 20 that extends into the body cavity when used during a closed surgical procedure. Elastic band 45 may be comprised of plastic, rubber or the like and is configured to operatively communicate with opening 46 to relieve excess pressure (in excess 50 mmHg) of during use. More particularly, when the pressure at distal end 16 of tube 20 reaches a predetermined threshold (e.g., greater than 50mmHg) elastic band 45 stretches to allow the over-pressurized gas to escape through opening 46. In this manner, pressure at the distal end 16 of tube 20 will never exceed 50 mmHg.

A second pressure relief mechanism 55 may also be included which operates to prevent over-insufflation of the body cavity during closed surgical procedures. Second pressure relief mechanism 55 includes a sleeve 56 dimensioned to fit about tube 20. Sleeve 56 has a proximal end 57 and a distal end 58. Proximal end 57 of sleeve 56 is received within distal end 14 of handpiece 11. An elastic band 59 operatively couples to the proximal end 57 of sleeve 56 about tube 20. Sleeve 56 extends from distal end 14 of handpiece 11 and preferably terminates proximal to distal end 16 of tube 20. In this manner, distal end 16 of tube 20 extends from handpiece 11 into the body cavity of the patient, thus fluidly connecting the body cavity with the outside environment. In the event the pressure within the cavity exceeds a specified amount, the high pressure may be released by elastic band 59 through proximal end 57 of sleeve 56.

A third pressure relief mechanism 65 may also be included as an alternate or redundant pressure relief valve. For example, valve 65 may be of any conventional valve design capable of releasing pressure over a specified amount. Valve 65 may be located along tube 20 with elongated body portion 15 of handpiece 11. It is envisioned that valve 65 may be configured such that when pressure is released through valve 65 an audible sound is heard. The sound emitted through valve 65 may range from a low hum to a high pitched whistle. The intensity and/or pitch of the sound may change as a function of the pressure being released through valve 65. Valve 65 may be connected to a sensor (not shown) located along tube 20 and electrically actuated to open when pressure within the cavity attains a predetermined level.

It is further envisioned that device 10 may be modified to include a humidifier. The humidifier may be formed integrally with the base or may be situated remotely. The humidifier would humidify the gas entering the body cavity, assuring that the cavity does not become dehydrated because of the additional gas circulating in the cavity.

It is also envisioned that the device 10 may be dimensioned as a pencil-like hand held device or as a pistol-like hand held device depending upon the particular surgical purpose. Moreover, device 10 may further be configured for robotic handling.

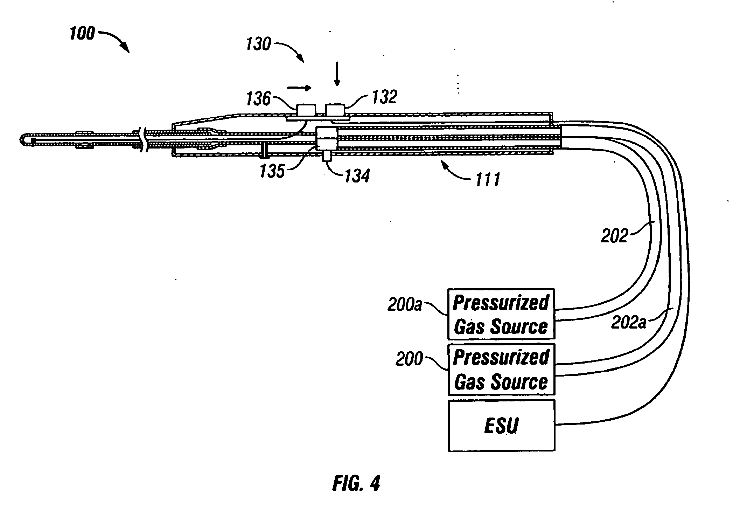

Referring now to FIG. 4, which does not show an embodiment of the present invention, electrosurgical device 100 is operably connected to a first and second pressurized source of gas 200, 200a via hoses 202, 202a respectively. Each of first and second gas sources 200, 200a may include the same or similar contents. Alternatively, each source 200, 200a may include different gases or different concentrations of the same gases. First and second gas sources 200, 200a may be pressurized to different levels such that each source dispenses its contents at a different rate. It is envisioned that either or both sources of gas 200, 200a may be treated, i.e. heated, ionize, prior to activation.

Electrosurgical device 100 includes a valve or diverter 135 configured for selectively alternating between first and second gas sources 200, 200a. Valve 135 includes a button or switch 134. Electrosurgical device 100 further includes an actuator assembly 130. Actuator 130 includes a slide switch or button 132. Actuator 130 operates in a manner similar to actuator 30 described above.

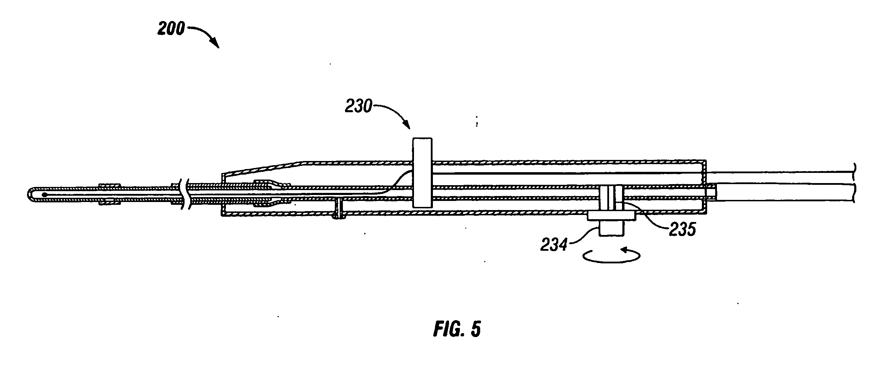

Turning now to FIG. 5, which does not show an embodiment of the present invention, electrosurgical instrument 200 includes a valve assembly 234 for regulating the flow of gas therethrough. Valve assembly 234 may be configured to dispense gas therethrough at various flow rates. Valve assembly 234 may also be configured to prevent the flow of gas therethrough. Electrosurgical instrument 200 further includes a button or switch 230 for activating an electrode mounted on a distal end thereof.

## Claims

1. A hand-held electrosurgical instrument comprising:
a housing (11)including a tube (20) extending therethrough, the tube having proximal and distal ends (16, 18) and a middle portion (17) therebetween, the proximal end being adapted to connect to at least a first source of gas (200) and the distal end being configured to deliver gas to a surgical site; and
an actuator (30) configured to selectively regulate the flow of gas through the tube, the actuator having at least a first position which allows a first predetermined rate of gas to flow through the tube for a first surgical purpose, and at least one subsequent position which allows at least one different rate of gas to flow through the tube for at least a second surgical purpose, **characterized in that**:
the actuator (30) includes a base (32) which operatively connects to the housing and which includes a stem (34) which extends therefrom, the stem of the actuator includes an opening (35) defined therethrough which communicates with the middle portion of the tube, the stem is positionable by the base from a position which closes off the flow of gas through the tube to a variety of intermediate positions which allow the flow of varying amounts of gas through the tube, whereby the depression of the base of the actuator causes movement of the stem and the opening, the more the base is depressed, the less obstructed is the flow of gas through the tube and upon complete depression of the base, the opening is completely aligned with the tube such that the pressurized gas is permitted to freely flow through tube.

2. The electrosurgical instrument of claim 1, further including an electrode assembly (25) configured to selectively ionize the gas for at least one of the first and second surgical purposes.

3. The electrosurgical instrument of any preceding claim, wherein the first surgical purpose includes at least one of dissection and coagulation and the second surgical purpose includes at least one of the other of dissection and coagulation.

4. The electrosurgical instrument of any one of the preceding claims, wherein the stem is spring biased to return to the closed-off position.

5. The electrosurgical instrument of any preceding claim, wherein the first source of gas is a portable cartridge.

6. The electrosurgical instrument of any preceding claim, wherein the first source of gas includes a cylinder containing pressurized ionizable gas.

7. The electrosurgical instrument of any preceding claim, further comprising at least one pressure relief mechanism (44, 55, 65) operatively coupled to the tube.

8. The electrosurgical instrument of claim 7, wherein the pressure relief mechanism (44) is located in close proximity to the patient.

9. The electrosurgical instrument of any preceding claim, further comprising a first pressure relief mechanism (44, 55, 65) operatively coupled to the tube for regulating gas pressure within the tube and a second pressure relief mechanism (55, 65) operative coupled to the hand-held device for regulating gas within the surgical site.

10. The electrosurgical instrument of any preceding claim, further comprising at least one pressure relief mechanism (44, 55, 65) operatively coupled to the tube which regulates the pressure flowing through the tube to below 50mmHg.

11. The electrosurgical instrument of any preceding claim, further comprising a first pressure relief mechanism (44, 55) operatively coupled to a first part of the tube and a second pressure relief mechanism (55, 65) operatively coupled to a second part of the tube proximal to the first pressure relief mechanism.

12. The electrosurgical instrument of claim 2, wherein the electrode is mounted in proximity to the distal end of the tube and operably connected to an electrosurgical generator.

13. The electrosurgical instrument of any of the preceding claims, comprising a humidifier for humidifying gas entering the surgical site.

14. An electrosurgical system for dissecting and coagulating tissue, comprising:
an electrosurgical generator;
at least a first source of gas; and
the electrosurgical instrument of any one of the preceding claims,
the proximal end of the housing being adapted to connect to the at least a first source of gas;
an electrode mounted in proximity to the distal end of the tube, the electrode operably connected to the electrosurgical generator and configured to selectively ionize the gas passing thereby.

## Patentansprüche

1. Tragbares elektrochirurgisches Instrument, umfassend:
ein Gehäuse (11) mit einer Röhre (20), die sich hierdurch erstreckt, wobei die Röhre ein proximales und distales Ende (16, 18) und einen Mittelabschnitt (17) dazwischen aufweist, wobei das proximale Ende dazu angepasst ist, mit zumindest einer ersten Gasquelle (200) verbunden zu sein und das distale Ende dazu ausgestaltet ist, Gas zu einer chirurgischen Lokalisation zuzuführen; und
einen Aktor (30), der dazu ausgestaltet ist, wahlweise den Gasstrom durch die Röhre zu regulieren, wobei der Aktor zumindest eine erste Position, die es einer ersten vorbestimmten Gasströmrate erlaubt, durch die Röhre für einen ersten chirurgischen Zweck zu strömen, und zumindest eine nachfolgende Position aufweist, die es zumindest einer abweichenden Gasströmrate ermöglicht, durch die Röhre für zumindest einen zweiten chirurgischen Zweck zu strömen, **dadurch gekennzeichnet, dass**:
der Aktor (30) eine Basis (32) aufweist, die mit dem Gehäuse wirkend verbunden ist und einen Stamm (34) aufweist, der sich von ihr erstreckt, wobei der Stamm des Aktors eine Öffnung (35) aufweist, die hierdurch definiert ist, die mit dem Mittelabschnitt der Röhre verbunden ist, wobei der Stamm durch die Basis aus einer Position, die den Gasstrom durch die Röhre abschließt, in eine Vielzahl von Zwischenpositionen positionierbar ist, die es der Strömung erlauben, verschiedene Mengen von Gas durch die Röhre strömen zu lassen, wobei das Eindrücken der Basis des Aktors eine Bewegung des Stamms und der Öffnung bewirkt, je mehr die Basis eingedrückt wird, desto weniger der Gasstrom durch die Röhre gehemmt ist, und bei vollständigem Eindrücken der Basis die Öffnung vollständig mit der Röhre fluchtet, so dass das unter Druck stehende Gas frei durch die Röhre strömen kann.

2. Elektrochirurgisches Instrument nach Anspruch 1, ferner umfassend eine Elektrodenanordnung (25), die dazu ausgestaltet ist, das Gas für den ersten und/oder zweiten chirurgischen Zweck wahlweise zu ionisieren.

3. Elektrochirurgisches Instrument nach einem vorhergehenden Anspruch, wobei der erste chirurgische Zweck ein Sezieren und/oder eine Koagulation umfasst und der zweite chirurgische Zweck das jeweils andere des Sezierens und/oder der Koagulation umfasst.

4. Elektrochirurgisches Instrument nach einem der vorhergehenden Ansprüche, wobei der Stamm durch eine Federkraft vorgespannt ist, um in die abgeschlossene Position zurückzukehren.

5. Elektrochirurgisches Instrument nach einem vorhergehenden Anspruch, wobei die erste Gasquelle eine tragbare Patrone ist.

6. Elektrochirurgisches Instrument nach einem vorhergehenden Anspruch, wobei die erste Gasquelle einen Zylinder aufweist, der unter Druck stehendes ionisierbares Gas enthält.

7. Elektrochirurgisches Instrument nach einem vorhergehenden Anspruch, ferner umfassend zumindest einen Druckablassmechanismus (44, 55, 65), der mit der Röhre wirkgekoppelt ist.

8. Elektrochirurgisches Instrument nach Anspruch 7, wobei der Druckablassmechanismus (44) in naher Umgebung des Patienten angeordnet ist.

9. Elektrochirurgisches Instrument nach einem vorhergehenden Anspruch, ferner umfassend einen ersten Druckablassmechanismus (44, 55, 65), der mit der Röhre wirkgekoppelt ist, um einen Gasdruck innerhalb der Röhre zu regulieren, und einen zweiten Druckablassmechanismus (55, 65), der mit der tragbaren Vorrichtung wirkgekoppelt ist, um Gas innerhalb der chirurgischen Lokalisation zu regulieren.

10. Elektrochirurgisches Instrument nach einem vorhergehenden Anspruch, ferner umfassend zumindest einen Druckablassmechanismus (44, 55, 65), der mit der Röhre wirkgekoppelt ist, die den Druck, der durch die Röhre strömt, auf unter 50mmHg reguliert.

11. Elektrochirurgisches Instrument nach einem vorhergehenden Anspruch, ferner umfassend einen ersten Druckablassmechanismus (44, 55), der mit einem ersten Teil der Röhre wirkverbunden ist, und einen zweiten Druckablassmechanismus (55, 65), der mit einem zweiten Teil der Röhre proximal zu dem ersten Druckablassmechanismus gekoppelt ist.

12. Elektrochirurgisches Instrument nach Anspruch 2, wobei die Elektrode in der Nähe des distalen Endes der Röhre und wirkverbunden mit einem elektrochirurgischen Generator angebracht ist.

13. Elektrochirurgisches Instrument nach einem der vorhergehenden Ansprüche, umfassend einen Befeuchter, um Gas zu befeuchten, das die chirurgische Lokalisation betritt.

14. Elektrochirurgisches System zum Sezieren und Koagulieren von Gewebe, umfassend:
einen elektrochirurgischen Generator;
zumindest eine erste Gasquelle; und
das elektrochirurgische Instrument nach einem der vorhergehenden Ansprüche,
wobei das proximale Ende des Gehäuses dazu angepasst ist, zumindest mit der ersten Gasquelle verbunden zu sein;
wobei eine Elektrode in der Nähe des distalen Endes der Röhre angebracht ist, wobei die Elektrode mit dem elektrochirurgischen Generator wirkverbunden und dazu ausgestaltet ist, das Gas, das an ihr vorbeiströmt, wahlweise zu ionisieren.

## Revendications

1. Instrument électrochirurgical tenu à la main comprenant:
un boîtier (11) comportant un tube (20) s'étendant à travers celui-ci, le tube ayant des extrémités proximale et distale (16, 18) et une portion médiane (17) entre celles-ci, l'extrémité proximale étant apte à être connectée à au moins une première source de gaz (200), et l'extrémité distale étant configurée pour délivrer du gaz à un site chirurgical; et
un actionneur (30) configuré pour régler sélectivement l'écoulement de gaz à travers le tube, l'actionneur ayant au moins une première position qui permet l'écoulement d'un premier taux de gaz prédéterminé à travers le tube dans un premier but chirurgical, et au moins une position suivante qui permet l'écoulement d'au moins un taux de gaz différent à travers le tube pendant au moins un deuxième but chirurgical, **caractérisé en ce que**:
l'actionneur (30) comporte une base (32) qui est reliée fonctionnellement au boîtier et qui comporte une tige (34) qui s'étend de celle-ci, la tige de l'actionneur présente une ouverture (35) définie à travers celle-ci qui communique avec la portion médiane du tube, la tige peut être positionnée par la base d'une position qui ferme l'écoulement de gaz à travers le tube à diverses positions intermédiaires qui permettent l'écoulement de diverses quantités de gaz à travers le tube, moyennant quoi l'enfoncement de la base de l'actionneur provoque un mouvement de la tige et l'ouverture, et plus la base est enfoncée, moins l'écoulement de gaz à travers le tube est obstruit et, lors de l'enfoncement complet de la base, l'ouverture est complètement alignée avec le tube de sorte que le gaz comprimé peut s'écouler librement à travers le tube.

2. Instrument électrochirurgical selon la revendication 1, comprenant en outre un ensemble d'électrodes (25) configuré pour ioniser sélectivement le gaz dans au moins un parmi le premier et le deuxième but chirurgical.

3. Instrument électrochirurgical selon l'une quelconque des revendications précédentes, dans lequel le premier but chirurgical comprend au moins une parmi la dissection et la coagulation, et le deuxième but chirurgical comprend au moins une de l'autre de la dissection et de la coagulation.

4. Instrument électrochirurgical selon l'une quelconque des revendications précédentes, dans lequel la tige est sollicitée par ressort pour retourner à la position fermée.

5. Instrument électrochirurgical selon l'une quelconque des revendications précédentes, dans lequel la première source de gaz est une cartouche portable.

6. Instrument électrochirurgical selon l'une quelconque des revendications précédentes, dans lequel la première source de gaz comporte un cylindre contenant du gaz ionisable sous pression.

7. Instrument électrochirurgical selon l'une quelconque des revendications précédentes, comprenant en outre au moins un mécanisme de détente de pression (44, 55, 65) fonctionnellement couplé au tube.

8. Instrument électrochirurgical selon la revendication 7, dans lequel le mécanisme de détente de pression (44) se situe à proximité étroite du patient.

9. Instrument électrochirurgical selon l'une quelconque des revendications précédentes, comprenant en outre un premier mécanisme de détente de pression (44, 55, 65) fonctionnellement couplé au tube pour réguler la pression du gaz dans le tube et un deuxième mécanisme de détente de pression (55, 65) fonctionnellement couplé au dispositif tenu à la main pour réguler le gaz dans le site chirurgical.

10. Instrument électrochirurgical selon l'une quelconque des revendications précédentes, comprenant en outre au moins un mécanisme de détente de pression (44, 55, 65) fonctionnellement couplé au tube qui régule la pression s'écoulant à travers le tube à en dessous de 50mmHg.

11. Instrument électrochirurgical selon l'une quelconque des revendications précédentes, comprenant en outre un premier mécanisme de détente de pression (44, 55) fonctionnellement couplé à une première partie du tube, et un deuxième mécanisme de détente de pression (55, 65) fonctionnellement couplé à une seconde partie du tube à proximité du premier mécanisme de détente de pression.

12. Instrument électrochirurgical selon la revendication 2, dans lequel l'électrode est installée à proximité de l'extrémité distale du tube et est fonctionnellement reliée à un générateur électrochirurgical.

13. Instrument électrochirurgical selon l'une quelconque des revendications précédentes, comprenant un humidificateur pour humidifier le gaz entrant dans le site chirurgical.

14. Système électrochirurgical pour la dissection et la coagulation du tissu, comprenant:
un générateur électrochirurgical;
au moins une première source de gaz; et
l'instrument électrochirurgical selon l'une quelconque des revendications précédentes,
l'extrémité proximale du boîtier étant apte à être connectée à la au moins une première source de gaz;
une électrode installée à proximité de l'extrémité distale du tube, l'électrode étant fonctionnellement reliée au générateur électrochirurgical et étant configurée pour ioniser sélectivement le gaz passant devant celle-ci.
